# EUROPEAN PATENT SPECIFICATION

(11) **EP 1 097 185 B1**
(45) Date of publication and mention of the grant of the patent: **09.10.2002**
(21) Application number: 99934651.3
(22) Date of filing: 10.07.1999
(51) Int. Cl.: C09J 7/04, A61F 13/02, A61L 15/24

(54) **USE OF A METALLOCENE-POLYETHYLENE NONWOVEN AS BACKING MATERIAL**
VERWENDUNG EINES METALLOCEN-POLYETHYLENVLIESSTOFFES ALS TRÄGERMATERIAL
UTILISATION DE METALLOCENE-POLYETHYLENE NON TISSE COMME MATERIAU DE RENFORCEMENT

(30) Priority: 10.07.1998 DE 19830864
(43) Date of publication of application: 09.05.2001
(73) Proprietor: Beiersdorf AG, 20253 Hamburg (DE)
(72) Inventor: ARAIDA, Yasurou, Kita-Ku, Osaka, 530-8611 (JP); LENZ, Dirk, D-20253 Hamburg (DE); TIMM, Jürgen, D-21218 Seevetal (DE)
(86) International application number: EP9904851
(87) International publication number: WO00002969

(56) References cited:
- WO-A-97/24222
- WO-A-97/42922
- DE-A- 19 628 268
- US-A- 5 681 301

## Description

The invention relates to the use of a metallocene-polyethylene nonwoven as backing material in particular for rapid dressings, prefabricated surgical dressings, roll plasters and/or tape dressings and adhesive tapes.

Plasters offerings high wear comfort are distinguished by their great conformity to contoured areas of the body, by their readiness to move with moving joints (the fingers, for example) and, moreover, by their permeability to water vapour or even to air.
As a class of materials, nonwovens are particularly suitable as plaster backings. They are generally permeable to air, less expensive to produce than wovens, provided that common fibre materials are used, and they impart valuable tactile properties.
A disadvantage found with the common nonwovens for plaster applications is that they have limited elasticity and so are not ideally suited to contoured areas of the body.

Nonwovens can be produced by diverse techniques: for example, by the dry process, the spinbonding process or wet processes. This is followed by a series of finishing steps. In some cases, chemical binders are used (dry-laid webs), which bring with them the disadvantage of unwanted chemicals in the vicinity of the wound when the plaster is used.

A particularly preferred process is the melt blown process, in which, inter alia, elastomeric polymers or polymer mixtures are melted in an extruder and processed further in a spinning process. This produces a nonwoven having very high mechanical strength and consisting of extremely fine fibres (< 10 µm in diameter). The elastic properties of the polymer are retained by the nonwoven; that is, the material when subjected to elongations which occur in the normal course of use of plasters (max. 50 % elongation) exhibits virtually complete recovery.
The thermoplastic polymers, block copolymers based on styrene-butadiene-styrene (Kraton), polyurethanes or polyether- and polyester-urethanes employed to date for this purpose have the disadvantage of in some cases a high price for the raw material or of unfavourable toxicological evaluation (for example, aromatic polyurethanes may give off aromatic amines).
Consolidation of these nonwovens takes place almost exclusively by thermal or mechanical means, so that the finished nonwoven does not come into contact with any further process chemicals or auxiliary chemicals in the course of its production. As a result, the materials produced by this process are particularly suitable for use in medical products such as plasters or dressing material.

EP 0 749 756 A2 discloses the production of nonwoven plasters based on polyester elastomers (more precisely, on a polybutylene terephthalate-polyether copolymer). The flexibility and extensibility of the material (> 600 %) are emphasised, and underline the special suitability of the material as a plaster backing.

EP 0 341 875 B1 discloses the use of an elastomeric nonwoven fibre web comprising melt-blown fibres having a diameter of less than 50 µm, with the limitation of a tensile strength of at least 30 g/2.5 cm/(g/m² basis weight). Thermoplastic elastomers described are polyurethanes, elastomeric polyesters, elastomeric polyamides, and A-B-A' block copolymers.

US 5 861 301 A discloses the use of a metallocene-polyethylene woven as a backing web, the said material can be provided with an adhesive.

WO 97 42922 A discloses the use of a nonwoven as a backing material. The nonwoven fabric can be made of polyolefines such as polyethylene, no use of a metallocene-polyethylene is made.

It is therefore the object of the invention to provide a backing material which avoids the disadvantages known from the prior art. This material should be inexpensive to produce and ecologically unobjectionable as well as offering pleasant wear comfort in use.

This object is achieved by the use of a backing material as set out in Claim 1. The subclaims relate to advantageous developments thereof.

The invention proposes using a metallocene-polyethylene nonwoven as a backing material, the said backing material being provided on at least one side with a self-adhesive coating.

The metallocene-polyethylene nonwoven preferably has the following properties:
- a basis weight of from 40 to 200 g/m², in particular from 60 to 120 g/m², and/or
- a thickness of from 0.1 to 0.6 mm, in particular from 0.2 to 0.5, and/or
- an ultimate tensile strength elongation lengthwise of from 300 to 700 % and/or
- an ultimate tensile strength elongation crosswise of from 250 to 550 %.

The fibres of the metallocene-polyethylene nonwoven preferably have a diameter of from 1 to 50 µm, in particular from 10 to 25 µm.

It has also been found advantageous if the metallocene-polyethylene nonwoven is additionally characterized by
- a load at 25 % elongation crosswise of from 0.7 to 4 N/cm and/or
- a load at 50 % elongation crosswise of from 0.85 to 6.0 N/cm and/or
- a load at 100 % elongation crosswise of from 0.6 to 8.0 N/cm and/or
- a plastic deformation after 5-fold elongation and release by 50 % of from 5 to 15 %.

It is possible to emboss the nonwovens. The embossing area is preferably between 2 and 40 %, more preferably 10 to 25 %. To emboss the nonwoven temperatures of 80 °C to 100 °C and pressures of 10 to 100 (kg/cm²) are used.

In one advantageous embodiment the polymer employed is a copolymer of ethylene and an α-olefin having a carbon number from C₄ to C₁₀, it being possible for the polyolefin to have a melt index of between 1 and 50 g/(10 min) and a density of from 860 to 900 kg/m³.

In another advantageous embodiment the metallocene-polyethylene polymer is blended with other polymers, for example LLDPE.

For the adhesive coating it is preferred to use commercially customary, pressure-sensitive adhesive compositions based on acrylate or on natural or synthetic rubber.

In addition, the reverse of the metallocene-polyethylene nonwoven may have been given an anti-adhesive treatment.

A new class of polymers for use as backing material is represented by the metallocene-polyethylenes, or single-site-catalysed PE grades.

These possess adjustable elastic properties and are priced very favourably in comparison with other thermoplastic elastomers.
Principle applications of the new materials are in connection with flexible packaging, hoses and tubes, cable sheathing, and injection mouldings. The elastomeric PE grades Affinity (Dow Chemical) and Engage (DuPont) consist of an octene-ethylene copolymer having a density of about 0.85 g/cm³.

Advantages of the use of m-PE over alternative thermoplastic elastomers, especially in connection with medical products, are in general as follows:
- material savings owing to better mechanical performance of the m-PE grades.
- low levels of residual catalyst in the polymer (up to 1000 times lower).
- lower raw-material price relative to other elastomeric polymers (Kraton, polyurethanes, polyesters, SIS).

As a result of this it is possible to produce a high-performance nonwoven for use in the plaster sector at a highly favourable price.

The present nonwoven material is manufactured on a conventional melt blown line, using elastomeric m-PE grades from DuPont or Dow Chemical, USA.
The materials have the physical properties indicated in Table 1.

The metallocene-polyethylene (mPE) is a polymer melt received by a die from an extruder and is further heated and extruded from a row of orifices as fine filaments while converging sheets of hot air (primary air) discharging from the die contact the filaments and by drag forces stretch the hot filaments to microsize. The filaments are collected in a random entangled pattern on a moving collector screen such as a rotating conveyer forming a nonwoven web of entangled microsized fibres.
The filaments freeze or solidify a short distance from the orifice aided by ambient air (secondary air).
Then the nonwoven web(sheet) is rolled up.

The m-PE nonwoven exhibits almost isotropic mechanical properties (identical values in lengthwise and crosswise directions). Even on multiple elongation by 50 % and subsequent contraction (5 cycles) a permanent plastic deformation of max. 20 % is obtained.

Coating the m-PE nonwoven with a pressure-sensitive adhesive composition yields pressure-sensitive adhesive (PSA) products which can conform outstandingly to contoured areas of the body and do not hinder any movement (for example, at the finger) by the body. The air-permeable material does not lead to any maceration, even after a prolonged period of wear. When the plaster is removed there is no splitting of the backing material, since the construction of the nonwoven gives it optimum cohesion. The outstanding properties of the nonwoven can be transferred to the plaster in an optimum manner.

The advantages set out above result in particularly advantageous applications of the PSA-coated m-PE nonwoven as rapid dressings, prefabricated surgical dressings, roll plasters and/or tape dressings.
As is generally the case with plasters, the rapid dressings or roll plasters can also be provided with a wound covering, which can in turn be lined with a release paper and sealed in sealing paper.

Furthermore, the nonwoven that has been treated to make it self-adhesive can also be employed with outstanding effect as an adhesive tape.

In the text below a use according to the invention is illustrated, using examples, without wishing unnecessarily to restrict the described invention.

### Examples

The following table gives an overview concerning the manufacturing of Ethylene-Octene Copolymer and the conditions. Furthermore, a comparative example with conventional PE-polymers was made. Table 1D contains the properties of the nonwovens.

**Table 1:**

| Manufacturing Condition of TOP-MB Nonwoven | | | | | | |
|---|---|---|---|---|---|---|
| A. Polymer | | | | | | |
| | Condition 1 | Condition 2 | Condition 3 | Condition 4 | Condition 5 | Control |
| Polymer | Ethylene-Octene Copolymer | | | | | LLDPE |
| Octene Content(%) | 13.5 | | 19.0 | 24.0 | 13.5 | |
| Melt Index (g/10min.) | 30 | | | | | 50 |
| Polymer Blend | Non | | | | LLDPE20% | |

| B. Melt Blown Condition | | | | | | |
|---|---|---|---|---|---|---|
| Nozzle Size (mm) | | | 0.1-10 | | | |
| Temp.of Extrusion(degC) | | | 250 - 350 | | | 320 |

| C. Embossing Condition | | | | | | |
|---|---|---|---|---|---|---|
| Embossing area(%) | | 10 | | | | |
| Temp.(degC) | | 85 | | | | |
| Pressure(kg/cm2) | | 35 | | | | |

| D. Nonwoven Properties | | | | | | |
|---|---|---|---|---|---|---|
| | Condition 1 | Condition 2 | Condition 3 | Condition 4 | Condition 5 | Control |
| Basis weight (g/m²) (EN 29073 P1) | 81.5 | 72.5 | 80.3 | 81.2 | 79.6 | 82.1 |
| Thickness(mm) (EN 29073 P2) | 0.35 | 0.32 | 0.43 | 0.42 | 0.43 | 0.55 |
| Tensile strength(N/cm) MD (EN 29073 P3) CD (EN 29073 P3) | 4.4 | 3.8 | 3.1 | 1.6 | 3.7 | 2.7 |
| | 2.4 | 2.0 | 2.2 | 1.0 | 3.3 | 1.4 |
| Elongation(%)(EN 29073 P2)MD CD | 480 | 443 | 486 | 396 | 258 | 80 |
| | 390 | 365 | 374 | 332 | 256 | 94 |
| 25%Modulus (%)CD | 0.85 | 0.75 | 0.96 | 0.35 | 1.76 | 0.84 |
| 50%Modulus(%)CD | 1.15 | 1.04 | 1.25 | 0.53 | 2.20 | 1.25 |
| 100%Modulus(%)CD | 1.50 | 1.34 | 1.57 | 0.76 | 2.61 | - |
| 50%Cycle Hysterysis(%) 5times* MD CD | 9 | 9 | 8 | 6 | 12 | 22 |
| | 10 | 12 | 9 | 6 | 12 | 22 |
| AirPermeability(cc/cm2/sec)** | 30 | 35 | 41 | 42 | 30 | 36 |
| Average Fiber Diameter (µm) | 17.4 | 18.2 | 16.9 | 18.4 | 15.1 | 15.3 |

| | | | | | | |
|---|---|---|---|---|---|---|
| *) Method of testing plastic deformation: Test strips of the sample are cut to a width of from 15 to 20 mm and are inserted with a clamped-in length of 100 mm into a tensile testing machine. The testing sequence consists of five successive loading and release phases with continuous load measurement at a maximum elongation of 50 %, the testing speed being 200 mm/min. The permanent elongation of the sample is given in %. | | | | | | |
| ** measured using the JIS 1096 | | | | | | |

Table 1D discloses the differences between the properties of the nonwovens made of mPE and the nonwovens made of conventional PE-polymers, especially after elongating the samples five times of 50 %. The mPE nonwovens show a permanent plastic deformation of max. 12 % compared with 22 % obtained with the conventional PE-polymers.

A nonwoven produced from elastomeric m-PE polymers (Dow Chemical, USA) by the melt blown process with subsequent thermal or water-jet consolidation and having a basis weight of 80 or 70 g/m², respectively, is coated with from 80 to 100 g/m² of a clinically tested rubber-based pressure-sensitive adhesive composition.

The good air permeability of the backing material permits air perforation of the applied composition by air brush during the application of the adhesive composition.

The metallocene-PE nonwovens treated with pressure-sensitive adhesive have the following physical properties:

**Table 2:**

| Physical properties of the coated metallocene-PE nonwovens | | | | |
|---|---|---|---|---|
| Parameter | Unit | md/cd | Adhesive film mPE nonwoven 80 g/m² | Adhesive film mPE nonwoven 70 g/m² |
| Basis weight (EN 29073 P1) | [g/m²] | | 162 | 149 |
| Thickness (EN 29073 P2) | [mm] | | 0.41 | 0.40 |
| Coating weight | [g/m²] | | 80 | 77 |
| Bond strength steel 180° | [N/cm] | | 5.4 | 5.0 |
| Air permeability (Gurley) | s [cm³/cm²*s] | | 1.9 (large plate) = 16.5 | 1.6 (large plate) = 19.6 |
| Load at 25 % elongation **) | [N/cm] | md | 2.03 | 1.50 |
| | | cd | 0.84 | 0.66 |
| Load at 50 % elongation **) | [N/cm] | md | 2.59 | 1.98 |
| | | cd | 1.18 | 1.01 |
| Load at 100 % elongation **) | [N/cm] | md | 3.15 | 2.48 |
| | | cd | 1.56 | 1.36 |

| | | | | |
|---|---|---|---|---|
| **) Conditions in accordance with EN 29073 P3 | | | | |

The coated web is cut into narrow rolls (3.75 cm, 8 cm) and processed further into roll plasters, tape products and, on converting machines, into plaster strips, by application of a wound pad.

The plaster products feature the following properties:
- very conformable backing material
- does not hinder movement at all, even on contoured parts of the body
- is air-permeable
- relatively non-aggressive adhesive compositions can be employed since, owing to the good elastic properties, lower shear forces occur
- hydrophobic surface of the material is water repellent

## Claims

1. Use of a metallocene-polyethylene nonwoven as backing material, the said backing material being provided on at least one side with a self-adhesive coating.

2. Use according to Claim 1, **characterized in that** the metallocene-polyethylene nonwoven
has a basis weight of from 40 to 200 g/m², in particular from 60 to 120 g/m², and/or
a thickness of from 0.1 to 0.6 mm.

3. Use according to Claim 1, **characterized in that** the metallocene-polyethylene nonwoven
has an ultimate tensile strength elongation lengthwise of from 300 to 700 % and/or an ultimate tensile strength elongation crosswise of from 250 to 550 %.

4. Use according to Claim 1, **characterized in that** the fibres of the metallocene-polyethylene nonwoven
have a diameter of from 1 to 50 µm,

5. Use according to Claim 1, **characterized in that** the polymer employed is a copolymer of ethylene and an α-olefin having a carbon number from C₄ to C₁₀, the polyolefin having a melt index of between 1 and 50 g/(10 min) and a density of from 860 to 900 kg/m³.

6. Use according to Claim 1, **characterized in that** the adhesive coating consists of a commercially customary, pressure-sensitive adhesive composition based on acrylate or on rubber.

7. Use according to Claim 1, **characterized in that** the reverse of the metallocene-polyethylene nonwoven has been given an anti-adhesive treatment.

8. Use according to at least one of Claims 1 to 7 as rapid dressings, prefabricated surgical dressings, roll plasters and/or tape dressings.

9. Use according to at least one of Claims 1 to 7 as adhesive tape.

## Patentansprüche

1. Verwendung eines Metallocen-Polyethylenvliesstoffes als Trägermaterial, wobei das genannte Trägermaterial mindestens auf einer Seite mit einer selbstklebenden Beschichtung versehen wird.

2. Verwendung im Einklang mit Anspruch 1, insofern charakterisiert, als der Metallocen-Polyethylenvliesstoff
eine Flächenmasse von 40 bis 200 g/m², im besonderen von 60 bis 120 g/m² und/oder
eine Stärke von 0,1 bis 0,6 mm aufweist.

3. Verwendung im Einklang mit Anspruch 1, insofern charakterisiert, als der Metallocen-Polyethylenvliesstoff
eine Zugfestigkeit mit einer Dehngrenze in Längsrichtung von 300 bis 700 % und/oder
eine Zugfestigkeit mit einer Dehngrenze in Querrichtung von 250 bis 550 % aufweist.

4. Verwendung im Einklang mit Anspruch 1, insofern charakterisiert, als die Fasern des Metallocen-Polyethylenvliesstoffes
einen Durchmesser von 1 bis 50 µm aufweisen.

5. Verwendung im Einklang mit Anspruch 1, insofern charakterisiert, als es sich bei dem verwendeten Polymer um ein Copolymer aus Ethylen und einem α-Olefin mit einer Kohlenstoffzahl von C₄ bis C₁₀ handelt, wobei das Polyolefin einen Schmelzindex zwischen 1 und 50 g/(10 min) und eine Dichte von 860 bis 900 kg/m³ aufweist.

6. Verwendung im Einklang mit Anspruch 1, insofern charakterisiert, als die Klebstoffbeschichtung aus einer handelsüblichen Kontaktklebstoffmischung auf der Basis von Acrylat oder Kautschuk besteht.

7. Verwendung im Einklang mit Anspruch 1, insofern charakterisiert, als die Rückseite des Metallocen-Polyethylenvliesstoffes eine adhäsionshemmende Behandlung erhält.

8. Verwendung im Einklang mit mindestens einem der Ansprüche 1 bis 7 als Schnellverbände, chirurgische Fertigverbände, Pflaster auf Rollen und/oder Streifenverbände.

9. Verwendung im Einklang mit mindestens einem der Ansprüche 1 bis 7 als Klebeband.

## Revendications

1. Utilisation d'un non tissé à base de métallocène-polyéthylène comme matériau de renforcement, ce matériau de renforcement étant couvert sur au moins une de ses faces d'un revêtement auto-adhésif.

2. Utilisation du matériau décrit en 1, **caractérisé par le fait que** le non tissé métallocène-polyéthylène a un poids de base compris entre 40 et 200 g/m², de préférence entre 60 et 120 g/m², et/ou une épaisseur comprise entre 0,1 et 0,6 mm.

3. Utilisation du matériau décrit en 1, **caractérisé par le fait que** le non tissé métallocène-polyéthylène affiche une résistance définitive à l'allongement dans le sens longitudinal comprise entre 300 et 700% et une résistance définitive à l'allongement dans le sens transversal comprise entre 250 et 550%.

4. Utilisation du matériau décrit en 1, **caractérisé par le fait que** les fibres du non tissé métallocène-polyéthylène ont un diamètre compris entre 1 et 50 µm.

5. Utilisation du matériau décrit en 1, **caractérisé par le fait que** le polymère employé est un copolymère d'éthylène et une α-oléfine ayant un nombre de carbones compris entre 4 et 10, la polyoléfine ayant un indice de fusion compris entre 1 et 50 g/ (10 min.) et une densité comprise entre 860 et 900 kg/m³.

6. Utilisation du matériau décrit en 1, **caractérisé par le fait que** le recouvrement adhésif consiste en un mélange adhésif à base d'acrylate ou de caoutchouc, sensible à la pression, et disponible dans le commerce.

7. Utilisation du matériau décrit en 1, **caractérisé par le fait que** la face arrière du non tissé à base de métallocène-polyéthylène a été recouverte d'un revêtement antiadhésif.

8. Utilisation conformément à un des points 1 à 7 au moins, en tant que pansements rapides, pansements chirurgicaux préfabriqués, sparadrap en rouleaux et/ou pansements adhésifs.

9. Utilisation conformément à un des points 1 à 7 au moins, en tant que bandes adhésives.
